# EUROPEAN PATENT APPLICATION

(11) **EP 4 697 681 A1**
(43) Date of publication of application: **18.02.2026**
(21) Application number: 24787952.1
(22) Date of filing: 02.04.2024
(51) Int. Cl.: H04L 69/08

(54) **REMOTE MEDICAL SYSTEM AND METHOD**

(30) Priority: 13.04.2023 CN 202310431600
(71) Applicant: Shenzhen Edge Medical Co., Ltd., Shenzhen, Guangdong 518000 (CN)
(72) Inventor: JIANG, Chengjun, Shenzhen, Guangdong 518000 (CN); GAO, Yuanqian, Shenzhen, Guangdong 518000 (CN)
(74) Representative: Biallo, Dario
(86) International application number: PCT/CN2024/085550
(87) International publication number: WO 2024/212847

(57) **Abstract**

A telemedicine system and a method thereof include: a first remote host receives an Ethercat message sent by a first master station, discards the Ethernet frame header of the Ethercat message, and obtains an Ethercat data frame; the first remote host encapsulates the Ethercat data frame to generate a UDP message including a UDP header and a custom protocol frame, and sends the UDP message to a second remote host, with a sending timestamp written into the custom protocol frame; a second remote host receives the UDP message, discards the UDP header of the UDP message, and obtains a data packet including a custom protocol frame and an Ethercat data frame; the second remote host obtains a valid data packet from the data packet based on the sending timestamp; the second remote host discards the custom protocol frame of the valid data packet, obtains an Ethercat data frame of the valid data packet, encapsulates the Ethercat data frame of the valid data packet to generate a target Ethercat message including a target Ethernet frame header, and sends the target Ethercat message to the second master station. This system enables low-cost and reliable remote surgery.

## Description

The present application claims priority to Chinese patent application with Application No. 202310431600.2, filed on April 13, 2023 to CNIPA, entitled "Telemedicine System and Method thereof," the entire contents of which are incorporated herein by reference.

### TECHNICAL FIELD

The present application relates to the field of medical devices, and more particularly to a telemedicine system and a method thereof.

### BACKGROUND

Minimally invasive surgery refers to a surgical procedure performed within a human body cavity using modern medical instruments such as laparoscopes and thoracoscopes and related equipment. Compared to traditional surgical procedures, minimally invasive surgery offers advantages such as less trauma, less pain, and faster recovery.

With technological advancements, minimally invasive surgical robot system technology has matured and is widely used. A surgical robot system comprises a master console and a slave operation device. The slave operation device includes multiple medical instruments with end-use instruments, including an imaging instrument with image end-use instruments and a surgical instrument with operation end-use instruments. The master console includes a display and an operation portion. The doctor manipulates the imaging instrument or surgical instrument within the field of view provided by the imaging instrument, as displayed on the monitor.

Recently, remote surgery technology has become increasingly popular. Using this technology, doctors can perform real-time surgeries on patients thousands of kilometers away, effectively alleviating the uneven distribution of medical resources between regions and reducing both time and financial costs for both doctors and patients. In order to ensure the reliable implementation of remote surgery, operators typically need to establish a dedicated network to enable communication between the master console and the slave operation device. However, this process is time-consuming and expensive. Therefore, there is an urgent need for a method to reliably perform remote surgery using the now widely available Internet.

### SUMMARY

Therefore, it is necessary to provide a telemedicine system and a control method thereof that can cost-effectively and reliably implement remote surgery.

The present application provides a telemedicine system, which includes:
a first surgical device, including a first master station;
a second surgical device, including a second master station;
a first remote host, connected to the first master station through an industrial Ethernet; and
a second remote host, connected to the second master station through the industrial Ethernet and remotely connected to the first remote host through an internet;
the first remote host is configured for:
   receiving an Ethercat message sent by the first master station, discarding an Ethernet frame header of the Ethercat message, and obtaining an Ethercat data frame; and
   encapsulating the Ethercat data frame to generate a UDP message comprising a UDP header and a custom protocol frame, sending the UDP message to the second remote host, and writing the custom protocol frame into a sending timestamp of the UDP message;
   the second remote host is configured for:
      receiving the UDP message, discarding the UDP header of the UDP message, and obtaining a data packet, wherein the data packet comprises the custom protocol frame and the Ethercat data frame;
      obtaining a valid data packet from the data packet based on the sending timestamp; and
      discarding a custom protocol frame of the valid data packet, obtaining an Ethercat data frame of the valid data packet, encapsulating the Ethercat data frame of the valid data packet to generate a target Ethercat message comprising a target Ethernet frame header, and sending the target Ethercat message to the second master station.

The present application also provides a method applied in a telemedicine system, the telemedicine system includes:
a first surgical device, including a first master station;
a second surgical device, including a second master station;
a first remote host, connected to the first master station through an industrial Ethernet; and
a second remote host, connected to the second master station through the industrial Ethernet and remotely connected to the first remote host through an internet;
the method includes:
   receiving, through the first remote host, an Ethercat message sent by the first master station, discarding an Ethernet frame header of the Ethercat message, and obtaining an Ethercat data frame; and
   encapsulating, through the first remote host, the Ethercat data frame to generate a UDP message comprising a UDP header and a custom protocol frame, sending the UDP message to the second remote host, and writing the custom protocol frame into a sending timestamp of the UDP message;
   receiving the UDP message, discarding the UDP header of the UDP message, and obtaining a data packet, through the second remote host, wherein the data packet comprises the custom protocol frame and the Ethercat data frame;
   obtaining a valid data packet from the data packet based on the sending timestamp through the second remote host; and
   discarding a custom protocol frame of the valid data packet, obtaining an Ethercat data frame of the valid data packet, encapsulating the Ethercat data frame of the valid data packet to generate a target Ethercat message comprising a target Ethernet frame header, and sending the target Ethercat message to the second master station, through the second remote host.

The present application also provides a computer-readable storage medium storing a computer program configured to be loaded by a processor and executed to implement the steps of the method described in any of the above embodiments.

The present application also provides a computer program product including computer instructions that, when executed on a computer, cause the computer to perform the method described in any of the above embodiments.

The telemedicine system and method of the present application have the following advantageous effects:
The first remote host converts the Ethercat message sent by the first master station into the UDP message for Internet transmission between the first remote host and the second remote host, and the second remote host converts a received UDP message back into an Ethercat message for transmission between the second remote host and the second master station, thereby enabling low-cost data exchange over the Internet. Furthermore, by encapsulating the custom protocol frame written with the sending timestamp in the UDP message and using the custom protocol frame to pre-process the obtained data packet, which includes the custom protocol and Ethercat data frames, to obtain the valid data packet, the reliability of subsequent processing based on the valid data packet is improved.

### BRIEF DESCRIPTION OF THE DRAWINGS

FIG. 1 is a schematic diagram of the principles of an embodiment of a telemedicine system of the present application;
FIG. 2 is a schematic diagram of a message processing process of an embodiment of a method of the present application;
FIG. 3 is a flow chart of an embodiment of a method of the present application;
FIG. 4 is a flow chart of another embodiment of a method of the present application;
FIG. 5 is a flow chart of a further embodiment of a method of the present application;
FIG. 6 is a flow chart of a furthermore embodiment of a method of the present application;
FIG. 7 is a structural schematic diagram of an embodiment of a telemedicine system of the present application.
FIG. 8 is a flow chart of an embodiment of a method of the present application;
FIG. 9 is a flow chart of another embodiment of a method of the present application;
FIG. 10 is a flow chart of a further embodiment of a method of the present application; and
FIG. 11 is a schematic diagram of the principles of a control device of a telemedicine system of the present application.

### DETAILED DESCRIPTION OF THE EMBODIMENTS

In order to facilitate understanding of the present application, the present application will be described more fully below with reference to the accompanying drawings. The drawings illustrate preferred embodiments of the present application. However, the present application can be implemented in many different forms and is not limited to the embodiments described herein. Rather, these embodiments are provided to facilitate a more thorough and comprehensive understanding of the disclosure of the present application.

It should be noted that when an element is referred to as being "disposed on" another element, it may be directly on the other element or there may also be an element centered. When an element is considered to be "coupled" to another element, it may be directly connected to the other element or there may be an element centered at the same time. When an element is considered to be "coupled" to another element, it may be directly coupled to the other element or there may be an element centered at the same time. The terms "vertical", "horizontal", "left", "right" and similar expressions used in the present application are for illustrative purposes only and do not represent the only implementation method. The terms "distal end" and "proximal end" used in the present application are used as directional words, which are commonly used terms in the field of interventional medical devices, where "distal end" refers to the end close to the patient during surgery and "proximal end" refers to the end away from the patient during surgery. The terms "first/second" and the like used in the present application refer to a component and a class of two or more components with common characteristics.

Unless otherwise defined, all technical and scientific terms used herein have the same meaning as commonly understood by those skilled in the art to which the present application pertains. The terms used herein are for the purpose of describing specific embodiments only and are not intended to limit the present application. The term "and/or" as used herein includes any and all combinations of one or more of the associated listed items. The term "each" as used herein includes one or more than two. The term "plurality" as used herein refers to two or more.

The present application provides a method for enabling low-cost and reliable bidirectional communication between two devices. For example, the method can be applied to a telemedicine system. In order to facilitate understanding, the present application uses the application of the method to a telemedicine system as an example.

In some embodiments, the telemedicine system includes a first surgical device and a second surgical device, which can utilize this method to achieve unidirectional and/or bidirectional communication. Because the data exchange process from the first surgical device to the second surgical device is substantially the same as the data exchange process from the second surgical device to the first surgical device, the present application uses the data exchange process from the first surgical device to the second surgical device as an example to illustrate this method.

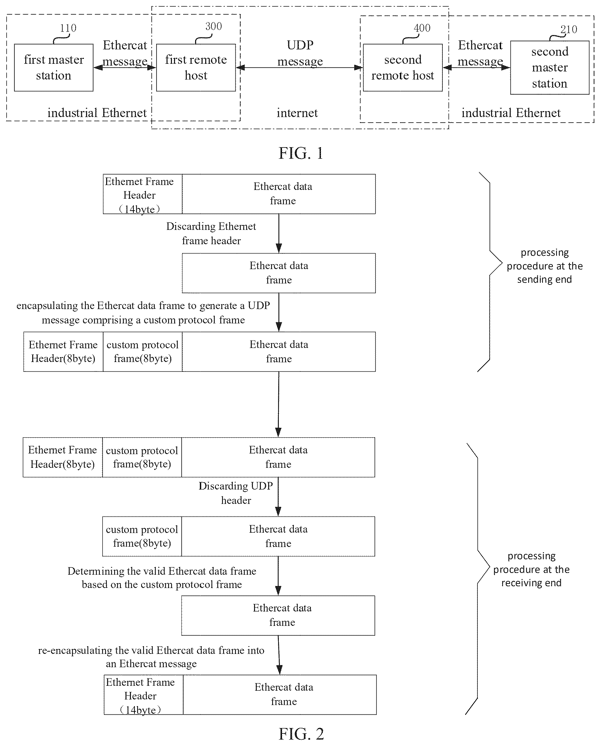

As shown in FIG. 1, the first surgical device includes a first master station 110, and the second surgical device includes a second master station 210. Both the first master station 110 and the second master station 210 can be Ethercat master stations, Both the first master station 110 and the second master station 210 are capable of capturing and processing the Ethercat messages. For example, both the first master station 110 and the second master station 210 can utilize Beckoff controllers.

In order to utilize the aforementioned method, the telemedicine system a further includes a first remote host 300 and a second remote host 400. The first remote host 300 can be integrated with the first surgical device or independent of the first surgical device; the second remote host 400 can be integrated with the second surgical device or independent of the second surgical device.

The first master station 110 and the first remote host 300 are connected through the industrial Ethernet, and the second master station 210 and the second remote host 400 are also connected through the industrial Ethernet. Furthermore, the first remote host 300 and the second remote host 400 are connected through the Internet. At least in the present application, the connection through the industrial Ethernet can be considered a local connection, while the connection through the Internet can be considered a remote connection.

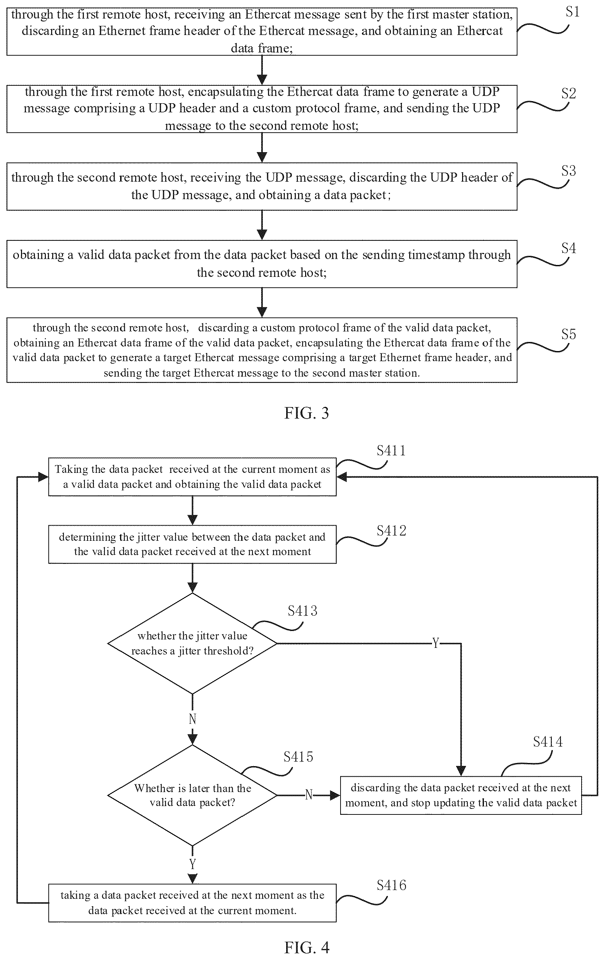

In some embodiments, as shown in FIGS. 2 and 3 , the method may include the following:
In a step S1: the first remote host receives an Ethercat message sent by the first master station, discards the Ethernet frame header of the Ethercat message, and obtains an Ethercat data frame.

The Ethercat message sent by the first master station 110 is an unprocessed Ethercat message, which can be understood as a source Ethercat message or an initial Ethercat message. Typically, an Ethercat message includes an Ethernet frame header, an Ethercat header, and Ethercat data. For ease of description, the present application defines the Ethercat header and Ethercat data together as an Ethercat data frame. After processing in the step S1, the Ethernet frame header is discarded from the Ethercat message, leaving only the Ethercat data frame. Since the Ethernet frame header is no longer needed, discarding the Ethernet frame header can avoid consuming network bandwidth. The Ethernet frame header includes a destination address segment and a source address segment. The destination address segment contains the physical address of the first remote host 300, and the source address segment contains the physical address of the first master station 110.

In a step S2, the first remote host encapsulates the Ethercat data frame to generate a UDP message including a UDP header and a custom protocol frame, and sends the UDP message to the second remote host.

The UDP header includes a source port segment and a destination port segment. The source port segment contains the communication port of the first remote host 300, and the destination port segment contains the communication port of the second remote host 400. In some embodiments, the communication ports of the first remote host 300 and the second remote host 400 can be determined based on NAT (Network Address Translation) traversal technology in WebRTC. After determining these two communication ports, the UDP message generated in the step S2 can be transparently transmitted between these two ports on the Internet using protocols such as IP, thereby resolving the issue of Ethercat messages being unable to be transmitted on the Internet.

The custom protocol frame contains the sending timestamp of the UDP message, which is written when the UDP message is sent to the second remote host 400. In some embodiments, the custom protocol frame includes 8 bytes.

In some embodiments, the principles of NAT traversal are briefly described as follows:
1. A persistent connection between the first remote host 300A and the second remote host 400B and the intermediary server S is established. In order to simplify the description, in this embodiment, A represents the first remote host 300A, B represents the second remote host, and S represents the intermediary server.
2. A sends a request to S, requesting a direct connection with B.
3. After receiving the request form A, S saves the public NAT-A IP address and port where A is located, and sends the public NAT-A IP address and port to B, requesting B to send a message to A.
4. After receiving the instruction from S, B sends a message to NAT-A, instructing NAT-B to open a port for NAT-A, allowing future messages from NAT-A to be received by B. Although B still cannot connect to A at this point, the traversal hole in the second remote host 400B has been established, allowing A to connect to device B through NAT-B.
5. After sending the message to NAT-A, B notifies S, "I'm ready." After receiving the ready message from B, S notifies A, instructing A to send a message to B.
6. After receiving the instruction from S, A sends a message to NAT-B. Because NAT-B opened a port for A in the step 4, the connection is established. Simultaneously, while A is sending the message to B, NAT-A also opens a port to receive a response from B. This completes the hole-establishing process between A and B, allowing A and B to communicate directly through NAT-A and NAT-B.

In a step S3: the second remote host receives the UDP message, discards the UDP header of the UDP message and obtains the data packet.

For ease of description, the present application defines the custom protocol frame and the Ethercat data frame as a data packet. After the UDP message is received by the second remote host 400, the UDP header can be discarded, retaining only the data packet that is required, as subsequent UDP messages are no longer applicable to the Industrial Ethernet.

In a step S4, the second remote host obtains a valid data packet from the data packet based on the sending timestamp.

Although the UDP message transmission over the Internet has various advantages, it also has significant disadvantages. For example, UDP message transmission over the Internet may be out of order. Therefore, in the step S4, the sending timestamp of the custom protocol frame can be used to pre-process the obtained data packet. For example, this can at least overcome the out-of-order problem and filter out valid data packets from the data packets, which at least meet the timing requirements.

In a step S5, the second remote host discards the custom protocol frame of the valid data packet, obtains an Ethercat data frame of the valid data packet, encapsulates the Ethercat data frame of the valid data packet to generate a target Ethercat message including a target Ethernet frame header, and sends the target Ethercat message to the second master station.

After pre-processing to obtain the valid data packet, the custom protocol frame with the sending timestamp is no longer used and thus can be discarded. After discarding the custom protocol frame, only the Ethercat data frame remains in the data packet. The critical data generated by the first surgical device written in the Ethercat data frame remains unchanged, thus providing high reliability. Furthermore, since the second master station 210 still operates on an industrial Ethernet network, the Ethercat data frame sent from the second remote host 400 to the second master station 210 needs to be re-encapsulated into an Ethercat message for transmission over the industrial Ethernet network. The Ethercat message re-obtained by encapsulating the Ethercat data frame of the valid data packet can be defined as the target Ethercat message.

The target Ethernet frame header included in the target Ethercat message includes a destination address segment and a source address segment. The destination address segment contains the physical address of the second master station 210, and the source address segment contains the physical address of the second remote host 400.

Through steps S1 to S5, that is, the first remote host 300 is used to convert the Ethercat message sent by the first master station 110 into a UDP message for Internet transmission between the first remote host 300 and the second remote host 400, and the second remote host 400 is used to reconvert the received UDP message into an Ethercat message for transmission between the second remote host 400 and the second master station 210, low-cost data exchange can be achieved over the Internet. Furthermore, by encapsulating a custom protocol frame with a sending timestamp in the UDP message and using the custom protocol frame to pre-process the obtained data packet including the custom protocol and Ethercat data frame to obtain a valid data packet, the reliability of subsequent processing based on the valid data packet is improved. Consequently, this method, when applied to a telemedicine system, offers significant advantages of low cost and reliability.

The step S4 performed by the second remote host 400 can be implemented in various ways.

In some embodiments, as shown in FIG. 4, the step S4 may include:
In a step S411, the data packet of the UDP message received at the current moment is initialized as a valid data packet and obtaining the valid data packet.

In the present application, the current moment generally refers to a moment that is currently occurring or has just arrived; the next moment is a future moment, generally refers to the moment that is closest and will soon arrive. The present application distinguishes the received UDP messages based on the current moment and the next moment to simplify the description of the above method or process.

In the step S411, the data packet corresponding to the UDP message at the current moment (obtained by parsing the UDP message at the current moment) is treated as a valid data packet and obtained for subsequent use.

In a step S412, the jitter value between the data packet and the valid data packet of the UDP message received at the next moment is determined based on the sending timestamp and receiving timestamp.

In the present application, for example, assume that at the sending end, the sending timestamp of the UDP message received at the current moment is st1, and the sending timestamp of the UDP message received at the next moment is st2; and assume that at the receiving end, the receiving timestamp of the UDP message received at the current moment is rt1, and the receiving timestamp of the UDP message received at the next moment is rt2. Since the sending time interval of the UDP messages at the two adjacent moments is Δts = *st*1 - st2 , and the receiving time interval is Δtr = *rt*1 - rt2, the jitter value between the two is Δt = Δtr - Δts. The second remote host 400 can obtain the receiving timestamp in real time when receiving the UDP message.

Since the Internet may have one or more network issues, such as network congestion and routing failures, if the interval between two UDP messages received at adjacent moments fluctuates, this can easily lead to jitter, such as intermittent speed fluctuations, when the first surgical device controls the second surgical device to execute motion control instructions. Therefore, determining the jitter value between the two UDP messages received at adjacent moments can be used to overcome this issues.

In a step S413, it is determined whether the jitter value reaches a jitter threshold.

The jitter threshold in the present application can be used to reflect the requirement of the telemedicine system for real-time data exchange. For example, the jitter threshold can reflect the maximum tolerance of the telemedicine system for real-time data exchange. The jitter threshold is expressed in time units, such as 10-80ms. The higher the real-time requirement, the lower the jitter threshold. In some embodiments, when the jitter value reaches the jitter threshold, the system may consider the jitter value excessive and unusable. When the jitter value does not reach the jitter threshold, the system may consider the jitter value to be small and tolerable, thus meeting the requirement. For example, "reach" can mean greater than, and correspondingly, "not reached" can mean less than or equal to. For another example, "reach" can mean greater than or equal to, and correspondingly, "not reached" can mean less than.

In a step S414, when the jitter value reaches the jitter threshold, the data packet of the UDP message received at the next moment is discarded.

The data packet of the UDP message received at the next moment that does not meet the jitter threshold cannot be used because it is intolerable, and therefore may be discarded.

After the step S414 has been executed, the process returns to the step S411. The transition from step S414 to step S411 can be understood as the end of one process in the method and the re-entry of a new process.

In a step S415, if the jitter value does not reach the jitter threshold, it is determined whether a sending timestamp of the data packet of the UDP message received at the next moment is later than the sending timestamp of the valid data packet based on the sending timestamp.

The jitter value not reaching the jitter threshold is only one of the necessary conditions for whether the data packet of the UDP message received at the next moment meets the usage requirements. In practice, it must also meet at least another necessary condition: specifically, its occurrence time sequence should not be earlier than the data packet of the UDP message received at the previous moment (i.e., the current moment). Therefore, the sending timestamps of two UDP messages received at adjacent moments can be used to determine the earlier or later occurrence time sequence between the two.

In a step S416, if the sending timestamp of the data packet of the UDP message received at the next moment is later than the sending timestamp of the valid data packet, a data packet of the UDP message received at the next moment is taken as the data packet of the UDP message received at the current moment.

As can be seen, in the step S416, the data packet of the UDP message received at the next moment is retained rather than discarded.

After the step S416 has been executed, returning to execute the step S411. The transition from step S416 to step S11 can be understood as retaining the data packet of the UDP message received at the next moment as a valid data packet and obtaining the valid data packet for subsequent use.

In a step S417, if the sending timestamp of the data packet of the UDP message received at the next moment is earlier than the sending timestamp of the valid data packet, the data packet of the UDP message received at the next moment is discarded.

The data packet of the UDP message received at the next moment that does not meet the transmission sequence cannot be used due to out-of-order issues and can therefore be discarded.

After the step S417 has been executed, returning to execute the step S411. The transition from step S417 to step S411 can be understood as the end of one process of the method and the re-entry of a new process.

Through the above steps S411-S417, the data packets of the received UDP message not only meet real-time requirements but also meet sequential requirements, thus achieving high reliability when applied to the telemedicine system.

The above steps S411-S417 are explained using an example.

In the first example, assuming that the UDP messages sent are a message 1 through a message7 in sequence, and their corresponding data packets are data packet 1 through data packet 7. Also assuming that the receiving order of the message 1 through message7 is: message 1, message 2, message 3, message 4, message 6, message 5, and message 7. Among them:
The UDP message received at the current moment is the message 1. At this time, its corresponding data packet 1 is initialized as a valid data packet 1 and the valid data packet 1 is obtained.

The UDP message received at the next moment is the message 2. At this time, if the jitter value between the message 2 (or data packet 2) and the message 1 (or data packet 1) meets the jitter threshold (i.e., the jitter value does not reach the jitter threshold), and it is clear that the message 2 is later than the message 1, the message 2 is regarded as the message at the current moment, and its corresponding data packet 2 is initialized as valid data packet 2 and the valid data packet 2 is obtained.

The UDP message received at the next moment is the message 3. At this time, if the jitter value between the message 3 and the message 2 meets the jitter threshold, and it is clear that the message 3 is later than the message 2, the message 3 is regarded as the message at the current moment, and its corresponding data packet 3 is initialized as valid data packet 3 and the valid data packet 3 is obtained.

The UDP message received at the next moment is the message 4. At this time, if the jitter value between the message 4 and the message 3 meets the jitter threshold, and it is clear that the message 4 is later than the message 3, the message 4 is treated as the message at the current time, and its corresponding data packet 4 is initialized as a valid data packet 4 and the valid data packet 4 is obtained.

The next UDP message received is the message 6. At this time, if the jitter value between the message 6 and the message 4 does not meet the jitter threshold, the message 6 corresponding to the data packet 6 is discarded.

The next UDP message received is the message 5. At this time, its corresponding the data packet 5 is initialized as a valid data packet 5 and the valid data packet 5 is obtained.

The next UDP message received is the message 7. At this time, if the jitter value between the message 7 and the message 5 meets the jitter threshold, and it is clear that the message 7 is later than the message 5, the message 7 is treated as the message at the current time, and its corresponding data packet 7 is initialized as a valid data packet 7 and the valid data packet 7 is obtained.

Furthermore, the valid data packets obtained above include valid data packets 1, 2, 3, 4, 5, and 7, and data packet 6 is discarded.

In the second example, assuming that the message 1 through message7 are sent in sequence, and their corresponding data packets are the data packet 1 through the data packet 7; and assuming that the receiving order of the message 1 through message7 is: message 2, message 1, message 3, message 4, message 6, message 7, and message 5.

The UDP message currently received is message 2. At this time, its corresponding data packet 2 is initialized as a valid data packet 2 and the valid data packet 2 is obtained.

The UDP message received next is the message 1. At this time, if the jitter value between the message 1 and the message 2 meets the jitter threshold, and it is clear that message 1 is earlier than message 2, and the data packet 1 corresponding to the message 1 is discarded.

The UDP message currently received is the message 3. At this time, its corresponding data packet 3 is initialized as a valid data packet 3 and the valid data packet 3 is obtained.

The next UDP message received is the message 4. At this time, if the jitter value between the message 4 and the message 3 does not meet the jitter threshold, the data packet 4 corresponding to message 4 is discarded.

The UDP message received at the current moment is the message 6. At this time, its corresponding data packet 6 is initialized as a valid data packet 6 and the valid data packet 6 is obtained;

The UDP message received at the next moment is the message 5. If the jitter value between the message 5 and the message 6 meets the jitter threshold, and it is clear that the message 5 is earlier than the message 6, the data packet 5 corresponding to the message 5 is discarded.

The UDP message received at the current moment is the message 7. At this time,the data packet 7 is initialized as a valid data packet 7 and and the valid data packet 7 is obtained.

The obtained valid data packets include valid data packets 2, 3, 6, and 7, while the data packets 1, 4, and 5 are discarded.

In the first and second examples above, the jitter value between two UDP messages received at adjacent times may or may not meet the jitter threshold. Through the processing of steps S411 to S417, valid data packets that meet the jitter and timing requirements can be screened out. In one embodiment, it is also feasible to first determine whether the sending timestamp of the data packet of the next UDP message is later than the sending timestamp of the valid data packet. If so, it is also feasible to then determine whether the jitter value between the data packet of the next UDP message received and the valid data packet reaches the jitter threshold.

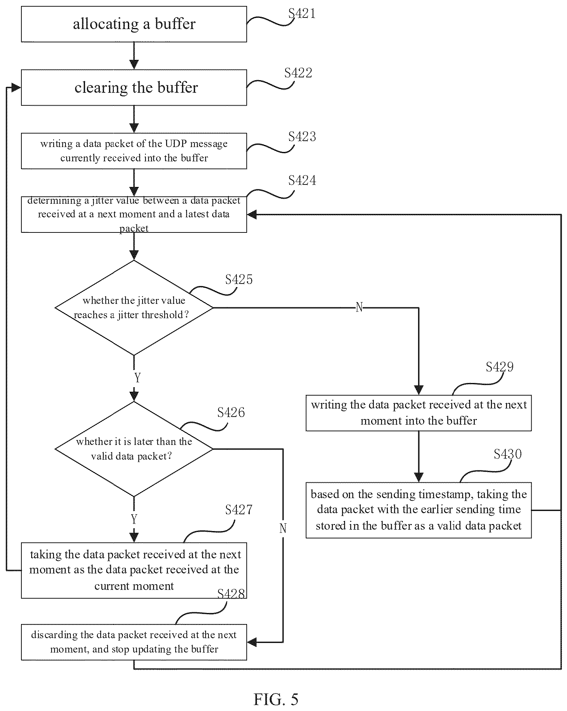

In some embodiments, as shown in FIG. 5, the step S4 may include:
In a step S421, a buffer is allocated.
In a step S422, the buffer is initialized.

Initializing the buffer also means clearing the buffer.

In a step S423, a data packet of the UDP message currently received is written into the buffer.

In a step S424, it is determined a jitter value between a data packet of the UDP message received at a next moment and the data packet stored in the buffer based on the sending timestamp and the receiving timestamp.

In a step S425, it is determined whether the jitter value reaches a jitter threshold.

In a step S426, when the jitter value reaches the jitter threshold, it is determined whether the data packet of the UDP message received at the next moment is later than the data packet stored in the buffer based on the sending timestamp.

In the step S426, if it is later than the data packet stored in the buffer, executing the step S427; if it is earlier than the data packet stored in the buffer, executing the step S428.

In a step S427, the data packet of the UDP message received at the next moment is taken as the data packet of the UDP message received at the current moment. After the step S427 has been executed, returning to execute the step S421.

In a step S428, the data packet of the UDP message received at the next moment is discarded. After the step step S428 has been executed, returning to execute the step S424.

In a step S429, if the jitter value does not reach the jitter threshold, the data packet of the UDP message received at the next moment is written into the buffer.

In a step S430, based on the sending timestamp, the data packet with the earlier sending time stored in the buffer is taken as a valid data packet and obtaining the valid data packet. After the step S430 has been executed, returning to execute the step S424. The buffer is primarily used to sort received out-of-order data packets, and then, based on the order within the buffer, the data packet with the earlier send time is taken as a valid data packet and the valid data packet is obtained.

The buffer is used for sorting will inevitably introduce latency, so the size of the buffer should not be too large, generally corresponding to the time range of two or three data packets. Data packets that fail to arrive beyond this time range are considered timed-out packets and are typically discarded to ensure the real-time performance of remote surgery within the telemedicine system.

The steps S421-S430 are described primarily based on the assumption that the buffer can store two data packets, and are illustrated below with an example.

Assuming that the UDP messages sent are a message 1 through a message 8, corresponding to a data packet 1 through a data packet 8. Assuming that the receiving order of the message 1 through the message 8 is: message 1, message 2, message 3, message 4, message 6, message 8, message 5, and message 7. [0, 0] represents an empty buffer, which contains the size of two data packets. In the embodiment:
The currently received message is the message 1. The data packet 1 is written into the buffer[0, 0], and the buffer is updated to [0, data packet 1].

The message received at the next moment is the message 2. If the jitter between the data packet 2 and the data packet 1 meets the jitter threshold, the data packet 2 is written into the buffer [0, data packet 1]. The buffer is updated (including sorting) to [data packet 2, data packet 1]. At this time, the earlier data packet 1 is retrieved, and the buffer is updated to [0, data packet 2]. The cycles for writing data packets to the buffer and retrieving data packets from the buffer are essentially the same. Furthermore, a single thread is typically used to write data packets into the buffer, using another thread to retrieve data packets from the buffer.

The message received at the next moment is the message 3. If the jitter value between the data packet 3 and the data packet 2 meets the jitter threshold, the data packet 3 is written into the buffer [0, data packet 2]. The buffer is updated to [data packet 3, data packet 2]. At this time, the earlier data packet 2 is retrieved, and the buffer is updated to [0, data packet 3].

The message received at the next moment is the message 4. If the jitter between the data packet 4 and the data packet 3 meets the jitter threshold, the data packet 4 is written into the buffer [0, data packet 3]. The buffer is updated to [data packet 4, data packet 3]. At this time, the earlier data packet 3 is retrieved, and the buffer is updated to [0, data packet 4].

The message received at the next moment is the message 6. If the jitter between the data packet 6 and the data packet 4 does not meet the jitter threshold, the buffer is initialized to [0, 0] and the data packet 6 is written into the buffer [0, 0] as the data packet 6 received at the current moment. The buffer is then updated to [0, data packet 6].

The message received at the next moment is the message 8. If the jitter between the data packet 8 and the data packet 6 does not meet the jitter threshold, the buffer is initialized to [0, 0], discarding the premature data packet 6 and writing data packet 8 into the buffer [0, 0] as the data packet 8 received at the current moment. The buffer is then updated to [0, 0]. The buffer is updated to [0, data packet 8].

The message received at the next moment is the message 5. If the jitter between the data packet 5 and the data packet 8 does not meet the jitter threshold, and the data packet 5 is clearly earlier than the data packet 8, the earlier data packet 5 is discarded, so the buffer remains unchanged at [0, data packet 8].

The message received at the next moment is the message 7. If the jitter between the data packet 7 and the data packet 8 meets the jitter threshold, the data packet 7 is written into the buffer [0, 0] as the data packet 7 received at the current moment. The buffer is updated to [data packet 8, data packet 7]. At this time, the earlier data packet 7 is removed, and the buffer is updated to [0, data packet 8].

Furthermore, the obtained valid data packets include valid data packets 1, 2, 3, 4, and 7; the data packets 1, 5, and 6 are discarded. Whether the data packet 8 is discarded is determined based on the message received immediately after data packet 7. Thus, the buffer can retain as many data packets as possible within a tolerable delay, preventing subsequent jitter, such as during motion control.

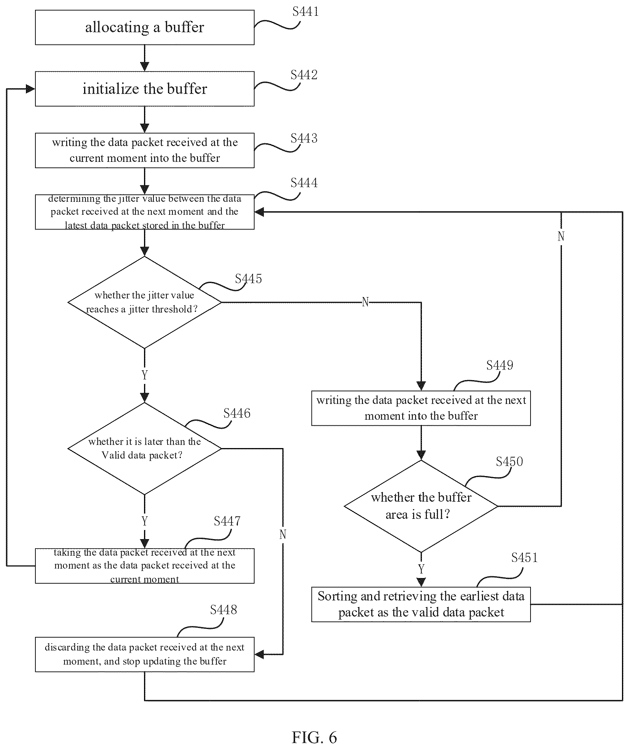

In some embodiments, as shown in FIG. 6, the step S4 may include:
In a step S441, a buffer is allocated;

The buffer includes a storage space for at least two data packet overheads. In some embodiments, the storage space in the buffer can store two or three data packets.
In a step S442, the buffer is initialized;
In a step S443, the data packet of the UDP message received at the current moment is written into the buffer;
In a step S444, the jitter value between the data packet of the UDP message received at the next moment and the data packet stored in the buffer is determined based on the sending timestamp and the receiving timestamp;
In a step S445, it is determined whether the jitter value reaches a jitter threshold.
In a step S446, when the jitter value reaches the jitter threshold, it is determined whether the data packet of the UDP message received at the next moment is later than the data packet stored in the buffer based on the sending timestamp.
In the step S446, if it is determined to be later than the data packet stored in the buffer, executing a step S447; if it is determined to be earlier than the data packet stored in the buffer, executing a step S448.
In a step S447, the data packet of the UDP message received at the next moment is taken as the data packet of the UDP message received at the current moment. After the step S447 has been executed, returning to execute the step S441;
In a step S448, the data packet of the UDP message received at the next moment is discarded. After the step S448 has been executed, returning to execute the step S444;
In a step S449, if the jitter value does not reach the jitter threshold, the data packet of the UDP message received at the next moment is written into the buffer;
In a step S450, it is determined whether the buffer is full.

In the step S450, if the buffer is not full, returning to execute the step S444; if the buffer is full, returning to execute the step S451.

In a step S451, based on the sending timestamp, the data packet of the UDP message with the earliest send time stored in the buffer is taken as a valid data packet and the valid data packet is obtained.

After the step S451 is executed, returning to execute the step S444.

Steps S441-S451 are primarily described based on the scenario where the buffer can store three data packets, and an example is provided below.

Assuming that the UDP messages sent are a message 1 to a message 8, corresponding to a data packet 1 to a data packet 8; and assuming that the receiving order of the message 1 to the message 8 is: message 1, message 2, message 3, message 4, message 7, message 5, and message 6. [0, 0, 0] represents an empty buffer, which contains the size of three data packets. Among them:
The currently received message is the message 1, the data packet 1 is written into the buffer [0, 0, 1], and the buffer is updated to [0, 0, data packet 1].

The message received at the next moment is the message 2. If the jitter between the data packet 2 and the data packet 1 meets the jitter threshold, the data packet 2 is written into the buffer [0, 0, data packet 1]. The buffer is updated (including the sorting) to [0, data packet 2, data packet 1]. At this time, the buffer is detected as not full, and the system continues to wait for the next message to be received.

The message received at the next moment is the message 3. If the jitter between the data packet 3 and the data packet 2 meets the jitter threshold (the data packet of the newly received message is compared with the data packets of the messages in the buffer with relatively later sending timestamps), the buffer is updated to [0, data packet 2, data packet 1], and the data packet 3 is written into the buffer [0, data packet 2, data packet 1], and the buffer is updated to [data packet 3, data packet 2, data packet 1]. At this time, the buffer is detected to be full, and the earlier packet, that is, the data packet 1, is retrieved, and the buffer is updated to [0, data packet 3, data packet 2]. Typically, one thread is used to write the data packet into the buffer, and another thread retrieves the data packet from the buffer.

The message received at the next moment is the message 4. If the jitter between the data packet 4 and the data packet 3 meets the jitter threshold, the data packet 4 is written into the buffer [0, data packet 3, data packet 2], the buffer is updated to [data packet 4, data packet 3, data packet 2]. At this time, the buffer is detected to be full, and the earlier packet, that is, the data packet 2, is retrieved, and the buffer is updated to [0, data packet 4, data packet 3].

The message received at the next moment is the message 7. If the jitter between the data packet 7 and the data packet 4 does not meet the jitter threshold, the buffer is cleared to [0, 0, 0], and the data packet 7 is written into the buffer [0, 0, 0] as the currently received data packet 7. The buffer is updated to [0, 0, data packet 7].

The message received at the next moment is the message 5. If the jitter between the data packet 5 and the data packet 7 meets the jitter threshold, the data packet 5 is written into the buffer [0, 0, data packet 7], and the buffer is updated to [0, data packet 7, data packet 5]. At this time, the buffer is detected as not full, and the system continues to wait for the next message to be received.

The message received at the next moment is the message 6. If the jitter between the data packet 6 and the data packet 7 meets the jitter threshold, the data packet 6 is written into the buffer [0, data packet 7, data packet 5]. The buffer is updated to [data packet 7, data packet 6, data packet 5]. At this time, the buffer is detected as full, and the older packet, that is, the data packet 5, is retrieved. The buffer is updated to [0, data packet 7, data packet 6], and the system continues to wait for the next message to be received.

Furthermore, the valid data packets obtained include valid data packets 1, 2, and 5, data packets 3 and 4 are discarded, and whether data packets 6 and 7 are discarded is determined based on the message received immediately after receiving the data packet 6. Generally, the larger the buffer, the longer the tolerable delay; that is, the jitter threshold can be determined based on the tolerable delay.

As can be seen, various implementations of the step S4 can obtain valid data packets that meet jitter and timing requirements, to improve reliability during remote surgery. In some embodiments, in the step S4, each valid data packet determined can be output and sent to the second master station.

In some embodiments, the telemedicine system includes a master console 100 and a slave operation device 200. The master console 100 is located at the doctor terminal for remote operation, and the slave operation device 200 is located at the patient terminal for performing surgery. The first surgical device includes one of the master console 100 and the slave operation device 200, and the second surgical device includes the another one of the master console 100 and the slave operation device 200.

In some embodiments, in an Ethercat message, a standard Ethernet frame header generally includes 14 bytes. Under normal circumstances, the master console 100 sends 2000 messages per second to the slave operation device 200, i.e., the interval between two messages is 0.5ms; corresponding to 2 to 3 messages, the buffering time is 1 to 1.5 milliseconds; the slave operation device 200 sends 1000 messages per second to the master console 100, i.e., the interval between two messages is 1 milliseconds; corresponding to 2 to 3 messages, the buffering time is 2 to 3 milliseconds; when transmitted on the Internet, since the Ethernet frame header is discarded (the standard Ethernet frame header includes 14 bytes), each message is reduced by 14 bytes, and the first direction from the master console 100 to the slave operation device 200 is reduced by 2000x14b=28kb, and the second direction from the slave operation device 200 to the master console 100 is reduced by 1000 x14b = 14kb bytes; if converted into bandwidth, when compared to normal, which has reduced 28x8 = 224kbps in the first direction, and 14x8 = 112kbps in the second direction, for a total reduction of 336kbps of network bandwidth. Even considering the addition of 8-byte custom protocol frames to obtain valid data packets when transmitting messages over the Internet, network bandwidth can still be significantly reduced. Therefore, the method and telemedicine system of the present application are suitable for lower network bandwidth environments.

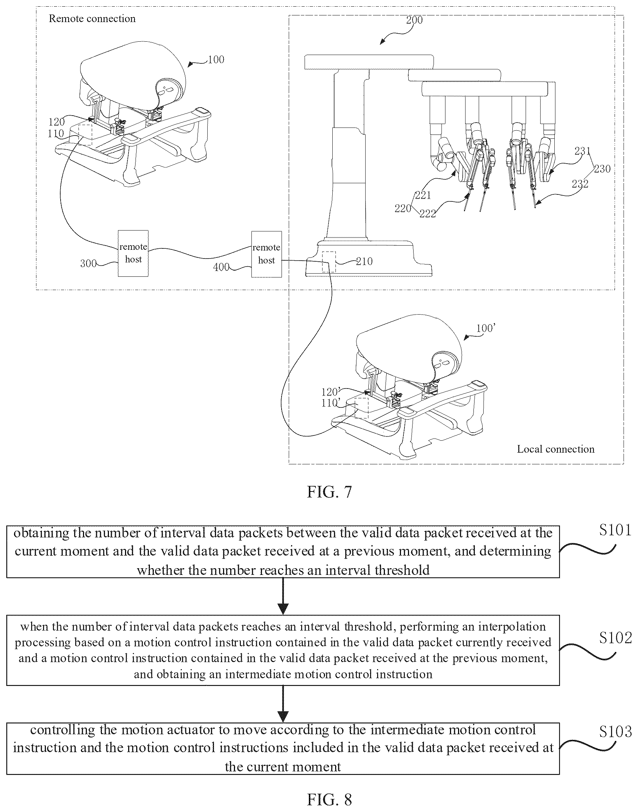

In some embodiments, as shown in FIG. 7, the first surgical device includes the master console 100, and the second surgical device includes the slave operation device 200. The master console 100 includes a motion input mechanism 120 for generating motion control instructions based on the input from a doctor. The motion input mechanism 120 is connected to the first master station 110 through the industrial Ethernet. The slave operation device 200 includes a motion actuator for moving according to the motion control instructions. The motion actuator is connected to the second master station 210 through the industrial Ethernet. The first master station 110 can be integrated into the master console 100 or independently of the master console 100; the second master station 210 can be integrated into the slave operation device 200 or independently of the slave operation device 200. In other embodiments, the second surgical device can also include an operating table for the patient to lie on or other devices that can be controlled during surgery.

For example, the motion input mechanism 120 includes a mechanical handle or a touch screen. For example, the motion actuator includes at least one manipulator assembly 220 (230), for example, the manipulator assembly 220 includes a manipulator 221 and a medical device mounted on the manipulator 221, the manipulator 221 includes a parallel four-bar linkage mechanism to define a remote motion center, and the medical device includes an imaging instrument 222 or a surgical instrument 232, which can move around the remote motion center and at least its end effector penetrates into the patient's body to perform imaging or surgery.

The Ethercat data frame of the Ethercat message generated by the master console 100 includes a motion control instruction, and the motion control instruction includes a position instruction and/or a posture instruction. The second master station 210 can be configured to perform the following steps of a method:
receiving a target Ethercat message, parsing the target Ethercat message to obtain a motion control instruction, and then controlling the motion actuator to move according to the motion control instruction.

In some embodiments, the second remote host 400 sends each valid data packet received to the second master station 210. In some embodiments, when the Ethercat data frame of the valid data packet includes the motion control instruction, as shown in FIG. 8, the second master station 210 may be configured to perform the following steps:
In a step S101, the number of interval data packets between the valid data packet received at the current moment and the valid data packet received at a previous moment is obtained, and it is determined whether the number reaches an interval threshold.

In a step S102, when the number of interval data packets reaches an interval threshold, an interpolation processing is performed based on a motion control instruction contained in the valid data packet currently received and a motion control instruction contained in the valid data packet received at the previous moment, and an intermediate motion control instruction is obtained.

The motion trajectory of the distal end of the motion actuator (i.e., the end effector of the medical device) can be planned based on the position and/or posture expressed by the motion control instructions corresponding to the valid data packets at the two moments. The motion trajectory is then time-segmented and interpolated to obtain the intermediate motion control instructions.

For example, the motion trajectory can be planned using an online planning method. For example, the motion trajectory can be designed as an S-shaped curve. Alternatively, the motion trajectory can be designed as a polynomial curve, such as a cubic curve or a quintic curve. For example, the motion trajectory can be designed as a trapezoidal curve. For example, a quintic curve with second-order continuity can be selected.

The number of interpolations can be determined based on requirements. For example, the number of interpolations can be equal to the number of interval data packets between the two moments. For another example, the number of interpolations can be greater or less than the number of interval data packets between the two moments. For example, in a process, if the valid data packet received at the current moment is the data packet 7, and the valid data packet received at the previous moment is the data packet 3, for example, 1, 2, 3, 4, or 5 intermediate motion control instructions can be obtained through interpolation.

In a step S103, the motion actuator is controlled to move according to the intermediate motion control instruction and the motion control instructions included in the valid data packet received at the current moment.

In the step S103, the motion control instructions included in the valid data packet received at the previous moment are pre-executed. Because the processing in step S4 filters out a large number of data packets that do not meet jitter and timing requirements between two adjacent moments, the motion trajectory of the distal end of the motion actuator can easily change abruptly. However, steps S101 to S103 can improve the smoothness of the motion trajectory of the distal end of the motion actuator.

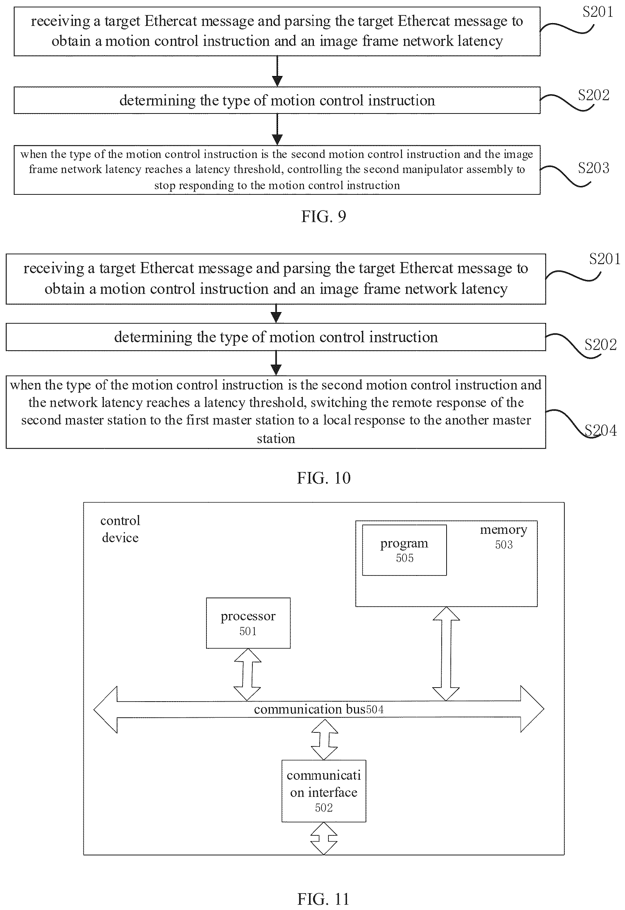

In some embodiments, as shown in FIG. 7, the motion actuator includes a first manipulator assembly 220 having an imaging instrument 222 and a second manipulator assembly 230 having a surgical instrument 232. The motion input mechanism 120 can generate motion control instructions for controlling the movement of the first manipulator assembly 220 and/or the second manipulator assembly 230. During remote surgery, the surgeon controls the movement of the imaging instrument 222 or the surgical instrument 232 while viewing the image captured by the surgical instrument 232 on the display screen of the master console 100. In some embodiments, the Ethercat data frames of the Ethercat messages generated by the master console 100 also include the network delay between the image frames captured by the surgical instrument 232 and the transmission from the second master station 210 to the first master station 110. As shown in FIG. 9, the second master station 210 can be configured to perform the following steps:
In a step S201, a target Ethercat message is received and the target Ethercat message is parsed to obtain a motion control instruction and an image frame network latency.
In a step S202, the type of motion control instruction is determined.

The motion control instruction includes a first motion control instruction for controlling the movement of the first manipulator assembly 220 and a second motion control instruction for controlling the movement of the second manipulator assembly 230. The first motion control instruction specifically controls the movement of the imaging instrument 222 to adjust the visual field, while the second motion control instruction specifically controls the movement of the surgical instrument 232 to perform surgical operations such as clamping, cutting, suturing, and suction.

The type of the motion control instruction can be determined, for example, based on the mapping relationship between the motion input mechanism 120 and the motion actuators 220 and 230.

In a step S203, when the type of the motion control instruction is the second motion control instruction and the image frame network latency reaches a latency threshold, the second manipulator assembly is controlled to stop responding to the motion control instruction.

The image frame network latency is affected by multiple factors, such as delays caused by encoding, transmission, and decoding. The delay threshold is exemplarily set to 80ms. If it is too long, it is easy to feel the hysteresis of the image and other problems. Among them, the network delay can be determined, for example, by calculating the time from the start of encoding to the completion of decoding. In the step S203, said controlling the second manipulator assembly 230 to stop responding to the control instruction includes multiple implementation methods, for example, the second main station 210 stops receiving or parsing the target Ethercat message, for example, the second main station 210 generates an emergency braking instruction to command the second manipulator assembly 230 to perform emergency braking, and for example, the second main station 210 generates an interrupt instruction to the second remote host 400 to control the second remote host 400 to disconnect from the first remote host 300. In some embodiments, the connection between the first remote host 300 and the second remote host 400 may not be interrupted, allowing the master console 100 to continue to receive and display image frames, but prohibiting the motion input mechanism 120 of the master console 100 from being enabled, so that the remote doctor can only provide guidance and not operate personally.

Through steps S201, S202, and S203, when the network latency of the remote surgery image frame does not meet the required requirements, the remote surgery is interrupted to ensure patient safety.

In some further embodiments, as shown in FIG. 7, the telemedicine system also includes another master console 100' located at the patient terminal for local operation by the doctor. The another master console 100' includes another motion input mechanism 120' and another master station 110' connected through the industrial Ethernet. The another master station 110' is locally connected to a second master station 210 through the industrial Ethernet. As shown in FIG. 10, the second master station 210 can be configured to perform the following steps:
In a step S201, a target Ethercat message is received and the target Ethercat message is parsed to obtain a motion control instruction and a network latency of the image frame;
In a step S202, the type of motion control instruction is determined; and
In a step S204, when the type of the motion control instruction is the second motion control instruction and the network latency reaches a latency threshold, the remote response of the second master station is switched to the first master station to a local response to the another master station.

There are various implementations for switching the remote response of the second master station 210 to the first master station 110 from a local response to the another master station 110'. For example, the remote connection between the first remote host 300 and the second remote host 400 can be terminated, and a local connection between the another master station 110' and the second master station 210 can be established. Alternatively, the remote connection between the first remote host 300 and the second remote host 400 can be maintained, but the motion input mechanism 120 of the master console 100 can be disabled. A local connection between the another master station 110' and the second master station 210 can then be established, and the motion input mechanism 120 of the other master console 100 can be enabled.

Through steps S201, S202, and S204, when the network latency of the remote surgery image frames does not meet the requirements, the remote surgery mode is switched to the local surgery mode, ensuring both patient safety and the continuation of the surgery.

In some embodiments, based on the description of the unidirectional data exchange process described above, where the slave operation device 200 serves as the first surgical device and the master console 100 serves as the second surgical device, the slave operation device 200 includes a status feedback mechanism connected to the first master station 110 through the industrial Ethernet, and the master console 100 includes a human-computer interaction device connected to the second master station 210 through the industrial Ethernet for providing status feedback in response to input from the status feedback mechanism.

For example, the status feedback mechanism includes, but is not limited to, various sensors for sensing the motion state or force conditions of the motion actuator, and various detection units for sensing software or hardware failures. For example, the human-computer interaction device includes audio-visual devices, such as display screens or speakers, for providing feedback on the motion state or software or hardware failures of the motion actuator, and a drive motor provided on the motion input mechanism 120 for providing feedback on force conditions.

The Ethercat data frames of the Ethercat messages generated by the slave operation device 200 contain status feedback instructions. The second master station 210 can be configured to perform the following steps:
receiving a target Ethercat message, parsing the target Ethercat message to obtain a status feedback instruction, and then controlling the human-computer interaction device to provide status feedback according to the status feedback instruction.

The present application also provides a control device for a telemedicine system. As shown in FIG. 11, the control system may include: a processor 501, a communication interface 502, a memory 503, and a communication bus 504.

The processor 501, the communication interface 502, and the memory 503 are communicated with each other through the communications bus 504.

The communications interface 502 is used to communicate with other devices, such as various sensors, rotating motors, solenoid valves, or other network elements such as clients or servers.

The processor 501 is used to execute a program 505, which may specifically perform the relevant steps in the above-mentioned method embodiment.

Specifically, the program 505 may include program code, which includes computer operating instructions.

The processor 505 may be a central processing unit (CPU), an Application Specific Integrated Circuit (ASIC), one or more integrated circuits configured to implement the embodiments of the present application, an FPGA, or a Graphics Processing Unit (GPU). The one or more processors included in the control device may be processors of the same type, such as one or more CPUs or one or more GPUs; or they may be processors of different types, such as one or more CPUs and one or more GPUs.

The memory 503 is used to store program 505. The memory 503 may include high-speed RAM memory, or may also include non-volatile memory, such as at least one disk storage.

The program 505 can specifically be configured to cause the processor 501 to perform the following operations:
receiving, through the first remote host, an Ethercat message sent by the first master station, discarding an Ethernet frame header of the Ethercat message, and obtaining an Ethercat data frame; and
encapsulating, through the first remote host, the Ethercat data frame to generate a UDP message comprising a UDP header and a custom protocol frame, sending the UDP message to the second remote host, and writing the custom protocol frame into a sending timestamp of the UDP message;
receiving the UDP message, discarding the UDP header of the UDP message, and obtaining a data packet, through the second remote host, wherein the data packet comprises the custom protocol frame and the Ethercat data frame;
obtaining a valid data packet from the data packet based on the sending timestamp through the second remote host; and
discarding a custom protocol frame of the valid data packet, obtaining an Ethercat data frame of the valid data packet, encapsulating the Ethercat data frame of the valid data packet to generate a target Ethercat message comprising a target Ethernet frame header, and sending the target Ethercat message to the second master station, through the second remote host.

The present application also provides a computer program product including computer instructions that, when executed on a computer, cause the computer to perform a method as described in any of the above embodiments.

The technical features of the above embodiments may be combined in any manner. In order to simplify the description, not all possible combinations of the technical features in the above embodiments are described. However, as long as no inconsistencies exist in the combination of these technical features, they should be considered within the scope of the specification.

The above embodiments merely represent a few implementation methods of the present application. While the descriptions are relatively specific and detailed, they should not be construed as limiting the scope of the present patent application. It should be noted that those skilled in the art could make various variations and improvements without departing from the spirit of the present application, and these variations and improvements are all within the scope of protection of the present application. Therefore, the scope of protection of this patent application shall be determined by the appended claims.

## Claims

1. A telemedicine system, comprising:
a first surgical device, comprising a first master station;
a second surgical device, comprising a second master station;
a first remote host, connected to the first master station through an industrial Ethernet; and
a second remote host, connected to the second master station through the industrial Ethernet and remotely connected to the first remote host through an internet;
wherein the first remote host is configured for:
receiving an Ethercat message sent by the first master station, discarding an Ethernet frame header of the Ethercat message, and obtaining an Ethercat data frame; and
encapsulating the Ethercat data frame to generate a UDP message comprising a UDP header and a custom protocol frame, sending the UDP message to the second remote host, and writing the custom protocol frame into a sending timestamp of the UDP message;
wherein the second remote host is configured for:
receiving the UDP message, discarding the UDP header of the UDP message, and obtaining a data packet, wherein the data packet comprises the custom protocol frame and the Ethercat data frame;
obtaining a valid data packet from the data packet based on the sending timestamp; and
discarding a custom protocol frame of the valid data packet, obtaining an Ethercat data frame of the valid data packet, encapsulating the Ethercat data frame of the valid data packet to generate a target Ethercat message comprising a target Ethernet frame header, and sending the target Ethercat message to the second master station.

2. The telemedicine system according to claim 1, **characterized in that**, said obtaining the valid data packet from the data packet based on the sending timestamp comprises:
initializing a data packet of the UDP message currently received as the valid data packet;
determining a jitter value between a data packet of the UDP message received at a next moment and the valid data packet;
determining whether the jitter value reaches a jitter threshold;
when the jitter value reaches the jitter threshold, discarding the data packet of the UDP message received at the next moment and returning to execute the step of initializing the data packet of the UDP message currently received as the valid data packet;
when the jitter value does not reach the jitter threshold, determining whether a sending timestamp of the data packet of the UDP message received at the next moment is later than the sending timestamp of the valid data packet;
if the sending timestamp of the data packet of the UDP message received at the next moment is later than the sending timestamp of the valid data packet, taking a data packet of the UDP message received at the next moment as the data packet of the UDP message received at the current moment, and returning to execute the step of initializing the data packet of the UDP message received at the current moment as the valid data packet; and
if the sending timestamp of the data packet of the UDP message received at the next moment is earlier than the sending timestamp of the valid data packet, discarding the data packet of the UDP message received at the next moment, and returning to the step of initializing the data packet of the UDP message received at the current moment as the valid data packet.

3. The telemedicine system according to claim 1, **characterized in that**, said obtaining the valid data packet from the data packet based on the sending timestamp comprises:
allocating a buffer, wherein the buffer comprises a storage space for two data packets;
initializing the buffer;
writing a data packet of the UDP message currently received into the buffer;
determining a jitter value between a data packet of the UDP message received at a next moment and the data packet stored in the buffer;
determining whether the jitter value reaches a jitter threshold;
when the jitter value reaches the jitter threshold, determining whether the sending timestamp of the data packet of the UDP message received at a next moment is later than the sending timestamp of the data packet stored in the buffer;
if the sending timestamp of the data packet of the UDP message received at the next moment is later than the sending timestamp of the data packet stored in the buffer, taking the data packet of the UDP message received at the next moment as the data packet of the UDP message received at the current moment, and returning to execute the step of initializing the buffer;
if the sending timestamp of the data packet of the UDP message received at the next moment is earlier than the sending timestamp of the data packet stored in the buffer, discarding the data packet of the UDP message received at the next moment, and returning to execute the step of determining the jitter value between the data packet of the UDP message received at the next moment and the data packet stored in the buffer;
when the jitter value does not reach the jitter threshold, writing the data packet of the UDP message received at the next moment into the buffer; and
taking the data packet with the earlier sending timestamp stored in the buffer as the valid data packet, and returning to execute the step of determining the jitter value between the data packet of the UDP message received at the next moment and the data packet stored in the buffer.

4. The telemedicine system according to claim 1, **characterized in that**, said obtaining the valid data packet from the data packet based on the sending timestamp comprises:
allocating a buffer, wherein the buffer comprises a storage space for two data packets;
initializing the buffer;
writing a data packet of the UDP message currently received into the buffer;
determining a jitter value between a data packet of the UDP message received at a next moment and the data packet stored in the buffer;
determining whether the jitter value reaches a jitter threshold;
when the jitter value reaches the jitter threshold, determining whether the sending timestamp of the data packet of the UDP message received at a next moment is later than the sending timestamp of the data packet stored in the buffer;
if the sending timestamp of the data packet of the UDP message received at the next moment is later than the sending timestamp of the data packet stored in the buffer, taking the data packet of the UDP message received at the next moment as the data packet of the UDP message received at the current moment, and returning to execute the step of initializing the buffer;
if the sending timestamp of the data packet of the UDP message received at the next moment is earlier than the sending timestamp of the data packet stored in the buffer, discarding the data packet of the UDP message received at the next moment, and returning to execute the step of determining the jitter value between the data packet of the UDP message received at the next moment and the data packet stored in the buffer;
when the jitter value does not reach the jitter threshold, writing the data packet of the UDP message received at the next moment into the buffer;
determining whether the buffer is full;
if the buffer is not full, returning to execute the step of determining the jitter value between the data packet of the UDP message received at the next moment and the data packet stored in the buffer; and
if the buffer is full, taking the data packet of the UDP message with an earliest sending timestamp stored in the buffer as the valid data packet and returning to execute the step of determining the jitter value between the data packet of the UDP message received at the next moment and the data packet stored in the buffer.

5. The telemedicine system according to claim 4, **characterized in that**, the buffer has a storage space for two or three data packets of the UDP message; and the custom protocol frame has 8 bytes of storage space.

6. The telemedicine system according to claim 1, **characterized in that**, the Ethernet frame header comprises a destination address segment writing into a physical address of the first remote host and a source address segment writing into a physical address of the first master station;
the target Ethernet frame header comprises a destination address segment writing into a physical address of the second master station and a source address segment writing into a physical address of the second remote host; and
the UDP header includes a source port segment writing into a communication port of the first remote host and a destination port segment writing into a communication port of the second remote host, wherein the communication port of the first remote host and the communication port of the second remote host are determined based on a network address translation technology.

7. The telemedicine system according to claim 1, **characterized in that**, the telemedicine system comprises a first master console and a slave operation device, wherein the first master console is located at a doctor terminal for remote operation, and the slave operation device is located at a patient terminal for performing surgery on a patient, wherein the first surgical device comprises one of the first master console and the slave operation device, and the second surgical device comprises another of the first master console and the slave operation device.

8. The telemedicine system according to claim 7, **characterized in that**, the first surgical device comprises the first master console, the second surgical device comprises the slave operation device, the first master console comprises a first motion input mechanism connected to the first master station through the industrial Ethernet, the slave operation device comprises a first manipulator assembly with an imaging instrument and a second manipulator assembly with a surgical instrument connected to the second master station through the industrial Ethernet, the first motion input mechanism generates a motion control instruction for controlling movement of the first manipulator assembly and/or the second manipulator assembly, and the Ethercat data frame comprises the motion control instruction and a network delay of an image frame captured by the imaging instrument from the second master station to the first master station.

9. The telemedicine system according to claim 8, **characterized in that**, the second master station is configured for:
receiving the target Ethercat message, and parsing the target Ethercat message to obtain the motion control instruction and the network delay; and
when a type of the motion control instruction is a motion control instruction configured for controlling the movement of the second manipulator assembly and the network delay reaches a delay threshold, controlling the second manipulator assembly to stop responding to the motion control instruction.

10. The telemedicine system according to claim 8, **characterized in that**, the telemedicine system further comprises a second master console located at the patient terminal for local operation by a doctor, the second master console comprises a second motion input mechanism and a third master station connected through the industrial Ethernet, the third master station is locally connected to the second master station through the industrial Ethernet; and the second master station is configured for:
when a type of the motion control instruction is a motion control instruction configured for controlling the movement of the second manipulator assembly and the network delay reaches a delay threshold, switching a remote response of the second master station to the first master station to a local response to the third master station.

11. The telemedicine system according to claim 8, **characterized in that**, the second master station is configured for:
obtaining a number of interval data packets between a valid data packet currently received and a valid data packet received at a previous moment;
when the number of interval data packets reaches an interval threshold, performing an interpolation processing based on a motion control instruction contained in the valid data packet currently received and a motion control instruction contained in the valid data packet received at the previous moment, and obtaining an intermediate motion control instruction to improve a smoothness of a motion trajectory of the first manipulator assembly and/or the second manipulator assembly.

12. The telemedicine system according to claim 11, **characterized in that**, said performing the interpolation processing based on the motion control instruction contained in the valid data packet currently received and the motion control instruction contained in the valid data packet received at the previous moment comprises:
planning a motion trajectory of at least one of the first manipulator assembly and the second manipulator assembly based on positions and/or postures corresponding to the motion control instructions contained in the valid data packet currently received and the motion control instructions contained in the valid data packet received at the previous moment, and performing a time-sliced interpolation on the motion trajectory to obtain the intermediate motion control instruction.

13. The telemedicine system of claim 12, **characterized in that**, the motion trajectory is planned online as one of an S-shaped curve, a polynomial curve, and a trapezoidal curve.

14. The telemedicine system of claim 11, **characterized in that**, a number of intermediate motion control instructions obtained through the interpolation processing is greater than or equal to the number of the interval data packets.

15. A method, applied to a telemedicine system, **characterized in that** the telemedicine system comprises:
a first surgical device, comprising a first master station;
a second surgical device, comprising a second master station;
a first remote host, connected to the first master station through an industrial Ethernet; and
a second remote host, connected to the second master station through the industrial Ethernet and remotely connected to the first remote host through an internet;
wherein the method comprises:
through the first remote host, receiving an Ethercat message sent by the first master station, discarding an Ethernet frame header of the Ethercat message, and obtaining an Ethercat data frame; and
through the first remote host, encapsulating the Ethercat data frame to generate a UDP message comprising a UDP header and a custom protocol frame, and sending the UDP message to the second remote host, wherein the custom protocol frame is written into a sending timestamp of the UDP message;
through the second remote host, receiving the UDP message, discarding the UDP header of the UDP message, and obtaining a data packet; wherein the data packet comprises the custom protocol frame and the Ethercat data frame;
obtaining a valid data packet from the data packet based on the sending timestamp through the second remote host; and
through the second remote host, discarding a custom protocol frame of the valid data packet, obtaining an Ethercat data frame of the valid data packet, encapsulating the Ethercat data frame of the valid data packet to generate a target Ethercat message comprising a target Ethernet frame header, and sending the target Ethercat message to the second master station .

16. A computer-readable storage medium, **characterized in that** the computer-readable storage medium stores a computer program, the computer program is configured to be loaded and executed by a processor to implement the steps of the method according to claim 15.
